# EUROPEAN PATENT SPECIFICATION

(11) **EP 3 273 970 B1**
(45) Date of publication and mention of the grant of the patent: **07.10.2020**
(21) Application number: 16773853.3
(22) Date of filing: 25.03.2016
(51) Int. Cl.: A61K 31/70, A61K 31/715, A61P 35/00, C08B 37/00

(54) **METHODS AND MATERIALS FOR TREATING CANCER**
VERFAHREN UND MATERIALIEN ZUR BEHANDLUNG VON KREBS
PROCÉDÉS ET MATÉRIELS POUR TRAITER LE CANCER

(30) Priority: 27.03.2015 US 201562139163 P
(43) Date of publication of application: 31.01.2018
(73) Proprietor: The Research Foundation for The State University of New York, Albany, NY 12207-2826 (US)
(72) Inventor: STEFANO, George B., Melville, New York 11747 (US); KREAM, Richard M., Huntington, New York 11743 (US); MANTIONE, Kirk J., Patchogue, New York 11772 (US)
(74) Representative: Kunz, Herbert
(86) International application number: PCT/US2016/024283
(87) International publication number: WO 2016/160590

(56) References cited:
- WO-A1-2015/041837
- WO-A2-2012/016050
- US-A1- 2011 077 217
- US-A1- 2015 065 451

## Description

### BACKGROUND

### 1. Technical Field

This document relates to materials for treating cancer. For example, this document relates to using compositions containing a potato polysaccharide preparation to reduce the number of cancer cells in a mammal. In some cases, this document relates to using compositions containing a potato polysaccharide preparation to reduce the number of cancer cells in a mammal, wherein the cancer cells express a v-Ki-ras2 Kirsten rat sarcoma viral oncogene homolog (KRAS) polypeptide.

### 2. Background Information

Potatoes are starchy, edible tubers obtained from potato plants and form an integral part of much of the world's food supply. In fact, potatoes are the fourth largest food crop in the world. The main potato species worldwide is *Solanum tuberosum.*

US 2011/0077217 A1 describes compositions for use in treating diseases such as cancer. The compositions described therein contain one or more polysaccharides in an admixture with one or more therapeutic agents, wherein the polysaccharide is selected from the group consisting of galactomannans (from *Cyamopsis tetragonolobus*), Arabinogalactan (from *Larix occidentalis*), Rhamnogalacturonan (from potato), Carrageenan (from *Eucheuma Seaweed*), and the Locust Bean Gun (from *Ceratonia siliqua*).

### SUMMARY

The present invention is defined by independent claim 1. The dependent claims depict additional embodiments of the invention.

This document provides materials for treating cancer. For example, this document provides compositions for use in reducing the number of cancer cells in a mammal, the compositions containing a potato polysaccharide preparation. In some cases, a composition containing a potato polysaccharide preparation provided herein can be used to reduce the number of cancer cells in a mammal, wherein the cancer cells express a KRAS polypeptide.

Having the ability to use a composition containing a potato polysaccharide preparation described herein to reduce the number of cancer cells in a mammal can provide clinicians and patients with an effective treatment regime for cancer.

This document also provides compositions (e.g., nutritional supplement compositions) that contain a potato polysaccharide preparation. For example, this document provides nutritional supplement compositions containing a potato polysaccharide preparation, methods for obtaining potato polysaccharide preparations, methods for making nutritional supplement compositions containing a potato polysaccharide preparation, and methods for increasing or decreasing expression of polypeptides involved with cancer.

In some cases, a composition containing a potato polysaccharide preparation provided herein can be used to decrease expression of a KRAS polypeptide and/or an oncogene polypeptide functionally interrelated with a KRAS polypeptide.

In some cases, the compositions provided herein (e.g., a nutritional supplement composition or a potato polysaccharide preparation provided herein) can be used to increase or decrease expression of polypeptides involved with cancer. For example, a composition containing a potato polysaccharide preparation provided herein or a potato polysaccharide preparation provided herein can be used to decrease expression of a KRAS polypeptide, a soc-2 suppressor of clear homolog (SOC2) polypeptide, an integrin-linked protein kinase (ILK) polypeptide, a heat shock 70 kDa protein (HSP9A) polypeptide, or a combination thereof.

In general, one aspect of this document features a composition for use in reducing the number of cancer cells in a mammal. The composition consists essentially of a potato polysaccharide preparation obtained from raw potatoes. The cancer cells are colorectal cancer cells, non-small-cell lung cancer cells, pancreatic cancer cells, liver cancer cells, or neuroblastoma cancer cells. The composition can reduce expression of a KRAS polypeptide. The composition can reduce expression of a SHOC2 polypeptide, an ILK polypeptide, or a HSP9A polypeptide. The mammal can be a human. The composition can consist essentially of the potato polysaccharide preparation in an amount that results in between 0.05 mg and 50 mg of the potato polysaccharide component of the potato polysaccharide preparation being administered to the mammal per kg of body weight of the mammal. The composition can consist essentially of between 1 mg and 100 mg of the potato polysaccharide preparation. The composition can consist essentially of between 6 mg and 20 mg of the potato polysaccharide preparation. The composition can consist essentially of between 1 mg and 100 mg of the potato polysaccharide component of the potato polysaccharide preparation. The composition can consist essentially of between 6 mg and 20 mg of the potato polysaccharide component of the potato polysaccharide preparation. The composition can be in the form of a tablet. The potato polysaccharide preparation can be in an amount that results in between 0.075 mg and 0.5 mg of the potato polysaccharide component of the potato polysaccharide preparation being administered to the mammal per kg of body weight of the mammal. At least about 80 percent of the potato polysaccharide preparation can be potato polysaccharide. At least about 90 percent of the potato polysaccharide preparation can be potato polysaccharide. At least about 95 percent of the potato polysaccharide preparation can be potato polysaccharide.

Unless otherwise defined, all technical and scientific terms used herein have the same meaning as commonly understood by one of ordinary skill in the art to which this invention pertains. Although methods and materials similar or equivalent to those described herein can be used in the practice or testing of the present invention, suitable methods and materials are described below. In case of conflict between the present specification and other references mentioned herein, the present specification, including definitions, will control. In addition, the materials, methods, and examples are illustrative only and not intended to be limiting.

Other features and advantages of the invention will be apparent from the following detailed description, and from the claims.

### DESCRIPTION OF DRAWINGS

Figure 1 is an HPLC chromatogram of a 10% ACN extract of raw potato (Russet Burbank).
Figure 2 is an HPLC chromatogram of collected and re-purified 3.5 minute peak material from a 10% ACN extract of raw potato shown in Figure 1.
Figure 3 is an LC/MS trace of 3.5 minute HPLC peak material.
Figure 4 is a full NMR spectrum of 3.5 minute HPLC peak material.
Figure 5 is an expanded NMR spectrum of 3.5 minute HPLC peak material.
Figure 6 is a total ion chromatogram of derivatized carbohydrate fragments of 3.5 minute HPLC peak material obtained from raw potato Russet Burbank).
Figure 7 is a fragmentation pattern of diacetamide. The peak fragmentation pattern is in the top panel, the compound library fragmentation match is in the bottom panel, and an overlay of the two is in the center panel.
Figure 8 is a fragmentation pattern of 3-acetoxy pyridine. The peak fragmentation pattern is in the top panel, the compound library fragmentation match is in the bottom panel, and an overlay of the two is in the center panel.
Figure 9 is a fragmentation pattern of 3,4-furan dimethanol, diacetate. The peak fragmentation pattern is in the top panel, the compound library fragmentation match is in the bottom panel, and an overlay of the two is in the center panel.
Figure 10 is a fragmentation pattern of 1,2,3-propanetriol diacetate. The peak fragmentation pattern is in the top panel, the compound library fragmentation match is in the bottom panel, and an overlay of the two is in the center panel.
Figure 11 is a fragmentation pattern of imidazole, 2-acetamino-5-methyl. The peak fragmentation pattern is in the top panel, the compound library fragmentation match is in the bottom panel, and an overlay of the two is in the center panel.
Figure 12 is a fragmentation pattern of 6,7-dihydro-5H-pyrrol[2,1,c][1,2,4] triazole-3-carboxylic acid. The peak fragmentation pattern is in the top panel, the compound library fragmentation match is in the bottom panel, and an overlay of the two is in the center panel.
Figure 13 is a fragmentation pattern of acetic acid, 1-(2-methyltetrazol-5-yl) ethenyl ester. The peak fragmentation pattern is in the top panel, the compound library fragmentation match is in the bottom panel, and an overlay of the two is in the center panel.
Figure 14 is a fragmentation pattern of 1,2,3,4-butanetriol, tetraacetate (isomer 1). The peak fragmentation pattern is in the top panel, the compound library fragmentation match is in the bottom panel, and an overlay of the two is in the center panel.
Figure 15 is a fragmentation pattern of 1,2,3,4-butanetriol, tetraacetate (isomer 2). The peak fragmentation pattern is in the top panel, the compound library fragmentation match is in the bottom panel, and an overlay of the two is in the center panel.
Figure 16 is a fragmentation pattern of pentaerythritol tetraacetate. The peak fragmentation pattern is in the top panel, the compound library fragmentation match is in the bottom panel, and an overlay of the two is in the center panel.
Figure 17 is a fragmentation pattern of 1,2,3,4,5-penta-o-acetyl-D-xylitol (isomer 1). The peak fragmentation pattern is in the top panel, the compound library fragmentation match is in the bottom panel, and an overlay of the two is in the center panel.
Figure 18 is a fragmentation pattern of 1,2,3,4,5-penta-o-acetyl-D-xylitol (isomer 2). The peak fragmentation pattern is in the top panel, the compound library fragmentation match is in the bottom panel, and an overlay of the two is in the center panel.
Figure 19 is a fragmentation pattern of 3,5-diacetoxy benzyl alcohol. The peak fragmentation pattern is in the top panel, the compound library fragmentation match is in the bottom panel, and an overlay of the two is in the center panel.
Figure 20 is a fragmentation pattern of β-D-galactopyranose, pentaacetate. The peak fragmentation pattern is in the top panel, the compound library fragmentation match is in the bottom panel, and an overlay of the two is in the center panel.
Figure 21 is a fragmentation pattern of D-mannitol hexaacetate. The peak fragmentation pattern is in the top panel, the compound library fragmentation match is in the bottom panel, and an overlay of the two is in the center panel.
Figure 22 is a fragmentation pattern of galacticol, hexaacetate. The peak fragmentation pattern is in the top panel, the compound library fragmentation match is in the bottom panel, and an overlay of the two is in the center panel.
Figure 23 is a fragmentation pattern of cyclohexane carboxylic acid, 1,2,4,5-tetrakis(acetoxy), (1α,3α,4α,5β)-(-). The peak fragmentation pattern is in the top panel, the compound library fragmentation match is in the bottom panel, and an overlay of the two is in the center panel.
Figure 24 is a fragmentation pattern of muco-inositol, hexaacetate. The peak fragmentation pattern is in the top panel, the compound library fragmentation match is in the bottom panel, and an overlay of the two is in the center panel.
Figure 25 is a fragmentation pattern of D-glucitol-hexaacetate. The peak fragmentation pattern is in the top panel, the compound library fragmentation match is in the bottom panel, and an overlay of the two is in the center panel.
Figure 26 is a fragmentation pattern of myo-inositol, hexaacetate. The peak fragmentation pattern is in the top panel, the compound library fragmentation match is in the bottom panel, and an overlay of the two is in the center panel.
Figure 27 is an HPLC chromatogram of a 10% ACN extract of raw Organic Yellow potato.
Figure 28 is an HPLC chromatogram of a 10% ACN extract of raw Purple potato.
Figure 29 is an HPLC chromatogram of a 10% ACN extract of raw Idaho Russet potato.
Figure 30 is an HPLC chromatogram of a 10% ACN extract of raw Yukon Gold potato.
Figure 31 is an HPLC chromatogram of a 10% ACN extract of raw sweet potato.
Figure 32 is an HPLC chromatogram of a 10% ACN extract of boiled Purple potato.
Figure 33 is an HPLC chromatogram of two pooled fraction collections from Idaho Russet potatoes.
Figure 34 is an HPLC chromatogram of fractions collections from 3 g of purple potatoes.
Figure 35 is a real time PCR amplification plot for KRAS demonstrating differences in threshold cycle numbers between potato polysaccharide preparation treated ZDF and untreated control ZDF rat liver tissue samples. The higher cycle number for the treated rat's tissue equates to a lower gene expression.
Figure 36 is a real time PCR amplification plot for ILK demonstrating differences in threshold cycle numbers (the point where the curve crosses the threshold) between potato polysaccharide preparation treated ZDF and untreated control ZDF rat liver tissue samples. The higher cycle number for the treated rat's tissue equates to a lower gene expression.
Figure 37 is a real time PCR amplification plot for SHOC2 demonstrating differences in threshold cycle numbers (the point where the curve crosses the threshold) between potato polysaccharide preparation treated ZDF and untreated control ZDF rat liver tissue samples. The higher cycle number for the treated rat's tissue equates to a lower gene expression.

### DETAILED DESCRIPTION

This document provides materials for treating cancer. For example, this document provides compositions for use in methods to reduce the number of cancer cells in a mammal. As described herein, a composition containing a potato polysaccharide preparation provided herein (e.g., a nutritional supplement composition provided herein) can be administered to any appropriate mammal to reduce the number of cancer cells within the mammal, to reduce tumor growth within the mammal, to increase survival time of the mammal, and/or to reduce the likelihood of metastasis within the mammal.

In some cases, this document provides materials related to treating mammals (e.g., humans) having cancer. Examples of mammals that can be treated as described herein include, without limitation, humans, monkeys, dogs, cats, cows, horses, pigs, ducks, rabbits, sheep, rats, and mice. Examples of cancers that can be treated as described herein include, without limitation, colorectal cancers, pancreatic cancers, non-small-cell lung cancers, liver cancers, or neuroblastoma cancers. A mammal can be identified as having cancer using any appropriate cancer diagnostic techniques.

The compositions provided herein (e.g., nutritional supplement compositions and potato polysaccharide preparations provided herein) can be used alone to reduce the number of cancer cells within a mammal.

Any appropriate route of administration (e.g., oral or parenteral administration) can be used to administer a composition containing a potato polysaccharide preparation provided herein (e.g., a nutritional supplement composition provided herein) to a mammal. For example, a composition containing a potato polysaccharide preparation provided herein can be administered orally. In some cases a composition containing a potato polysaccharide preparation provided herein can be administered by injection.

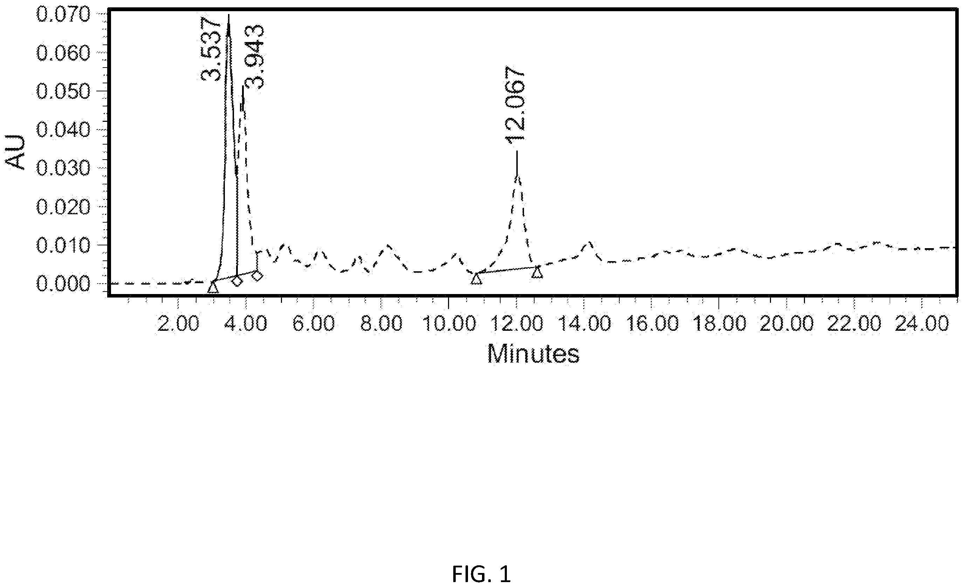

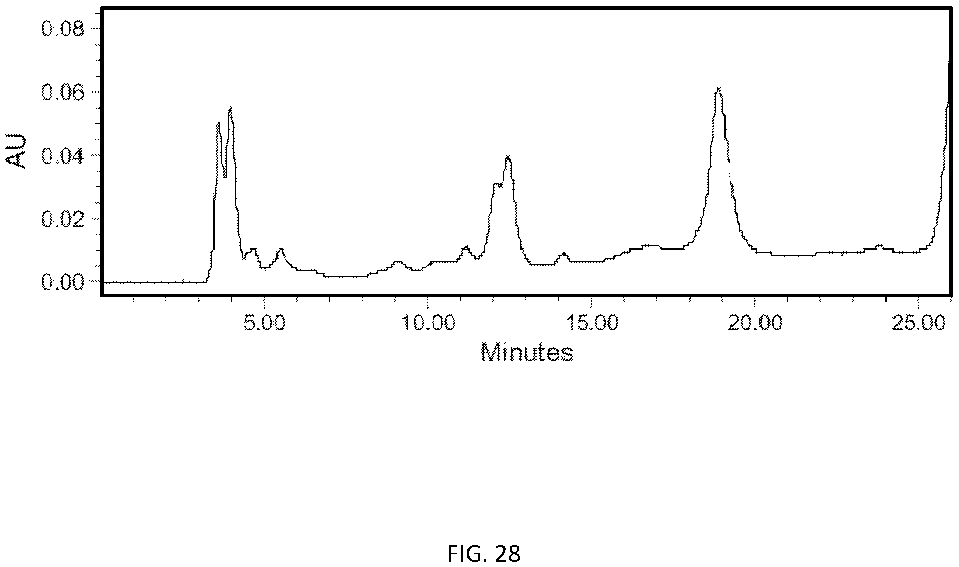

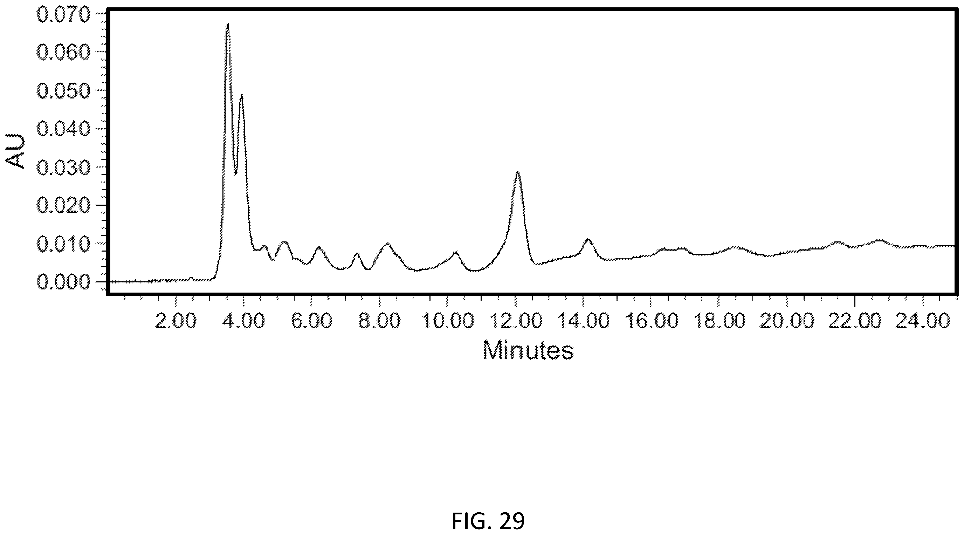

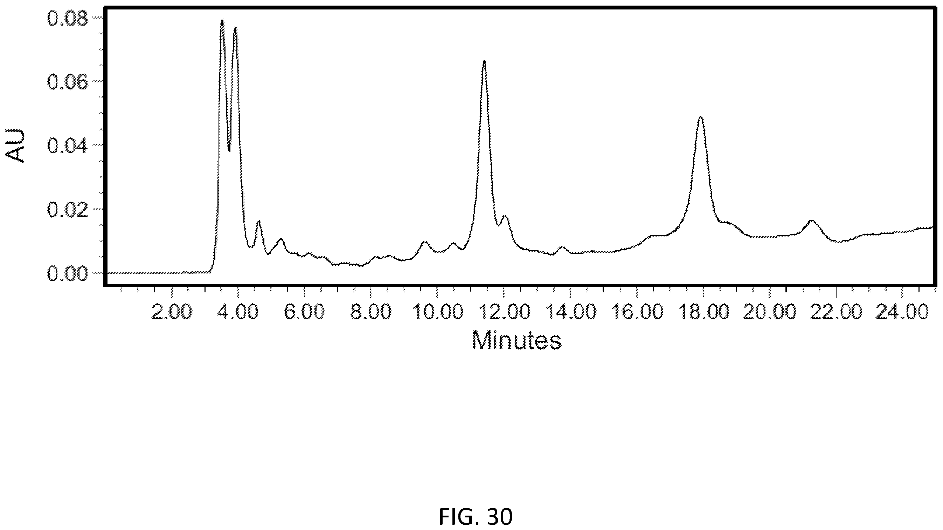

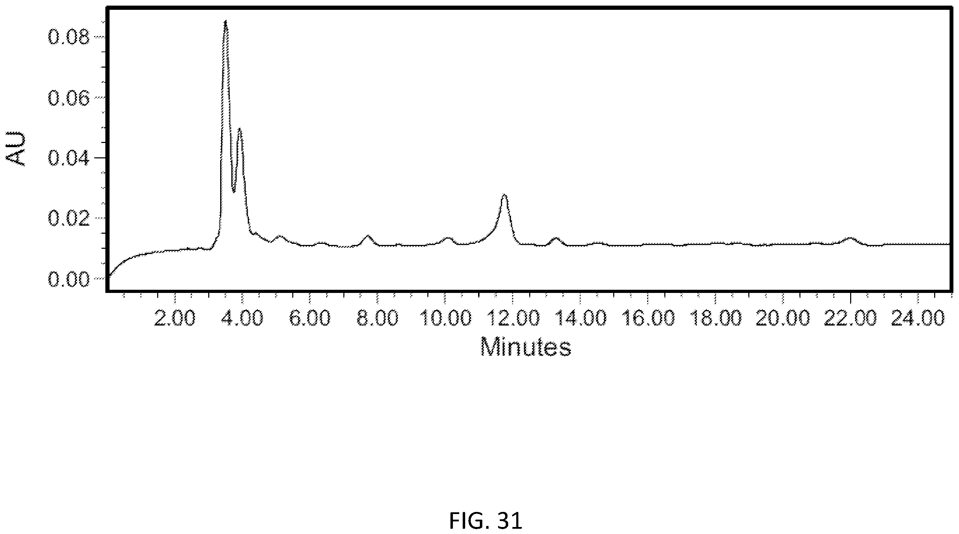

A composition provided herein (e.g., a nutritional supplement composition) can include one or more potato polysaccharide preparations. A potato polysaccharide preparation can be a preparation that is obtained from a water extract of potato and that contains polysaccharide material having the ability to be eluted from a C18 cartridge (e.g., a Sep-Pak Plus C-18 cartridge) with 10% acetonitrile. In some cases, a potato polysaccharide preparation can be a preparation that is obtained from potato and that contains polysaccharide material having HPLC characteristics of that of the peak eluted at 3.5 minutes as described in Example 1 (see, also, Figures 1, 2, and 27-33). In some cases, a polysaccharide of a potato polysaccharide preparation provided herein can be a polar, water-soluble polysaccharide. In some cases, a polysaccharide of a potato polysaccharide preparation provided herein can be a highly substituted complex xyloglucan material.

In some cases, a potato polysaccharide preparation can be a preparation that is obtained from potato and that contains polysaccharide material that, when derivatized, results in at least the following acylated carbohydrates as assessed using GC/MS: (a) myo-inositol (set to 1X to serve as an internal standard), (b) glucose at about 40X to about 60X the myo-inositol content (e.g., glucose at about 50X the myo-inositol content), (c) xylose at about 10X to about 20X the myo-inositol content (e.g., xylose at about 15X the myo-inositol content), (d) mannose at about 5X to about 15X the myo-inositol content (e.g., mannose at about 10X the myo-inositol content), and (e) galactose at about 3X to about 7X the myo-inositol content (e.g., galactose at about 5X the myo-inositol content). The derivatization procedure can include forming a dry residue of the polysaccharide material that is then hydrolyzed using trifluoroacetic acid. The resulting material is then reduced using sodium borohydride, and after borate removal, the end product is acylated using acetic anhydride and pyridine. The end products of the reaction are then injected directly on GC/MS to identify the acylated carbohydrates.

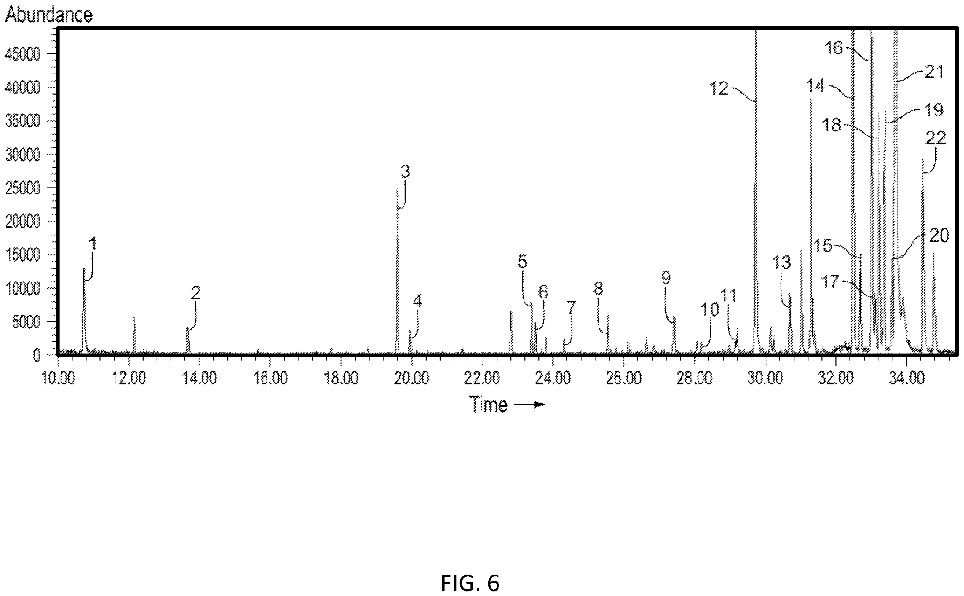

In some cases, a potato polysaccharide preparation can be a preparation that is obtained from potato and that contains polysaccharide material that, when derivatized and assessed using GC/MS, results in at least four major components (3,4-furan dimethanol, diacetate; 1,2,3,4,5-penta-o-acetyl-D-xylitol (isomer 1); 3,5-diacetoxy-benzyl alcohol; and D-glucitol-hexaacetate). See, e.g., Example 1. In some cases, a potato polysaccharide preparation can be a preparation that is obtained from potato and that contains polysaccharide material that, when derivatized and assessed using GC/MS, results in the compounds listed in Table 1 or results in the profile shown in Figure 6.

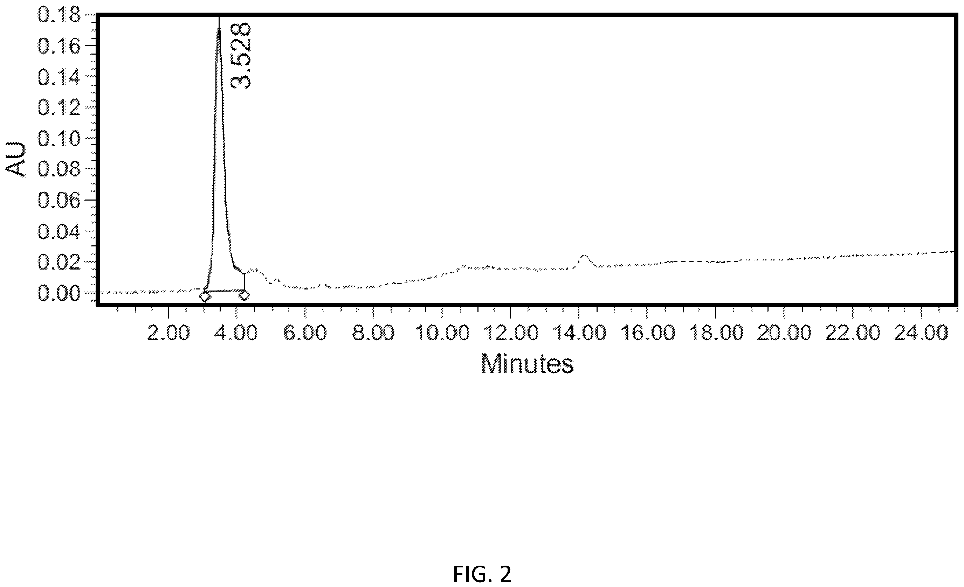

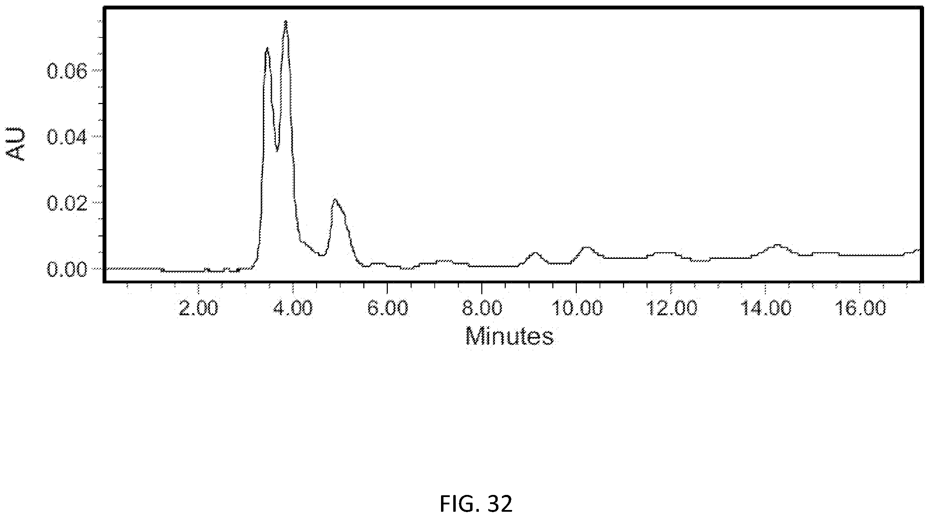

In some cases, a potato polysaccharide preparation provided herein can be a substantially pure potato polysaccharide preparation. Typically, a substantially pure potato polysaccharide preparation is a preparation that contains a single peak of material (e.g., a single peak of polysaccharide material) when assessed using, for example, HPLC (see, e.g., Figures 2 and 32). In some cases, greater than 60, 70, 75, 80, 85, 90, 95, or 99 percent of a potato polysaccharide preparation provided herein can be polysaccharide material obtained from a potato.

Any appropriate potato species or variety can be used to obtain a potato polysaccharide preparation provided herein. For example, Solanum tuberosum, Ipomoea batatas, S. acaule, S. bukasovii, S. leptophyes, S. megistacrolobum, S. commersonii, or S. infundibuliforme can be used to obtain a potato polysaccharide preparation provided herein. In some cases, potato varieties of S. tunerosum such as Organic Yellow, Purple or blue varieties, Cream of the Crop, Adirondack Blue, Adirondack Red, Agata, Almond, Andes Gold, Andes Sun, Apline, Alturas, Amandine, Annabelle, Anya, Arran Victory, Atlantic, Avalanche, Bamberg, Bannock Russet, Belle de Fontenay, BF-15, Bildtstar, Bintje, Blazer Russet, Blue Congo, Bonnotte, British Queens, Cabritas, Camota, Canela Russet, Cara, Carola, Chelina, Chiloe, Cielo, Clavela Blanca, Desiree, Estima, Fianna, Fingerling, Flava, German Butterball, Golden Wonder, Goldrush, Home Guard, Innovator, Irish Cobbler, Jersey Royal, Kennebec, Kerr's Pink, Kestrel, Keuka Gold, King Edward, Kipfler, Lady Balfour, Langlade, Linda, Marcy, Marfona, Maris Piper, Marquis, Megachip, Monalisa, Nicola, Pachacoña, Pike, Pink Eye, Pink Fir Apple, Primura, Ranger Russet, Ratte, Record, Red LaSoda, Red Norland, Red Pontiac, Rooster, Russet Burbank, Russet Norkotah, Selma, Shepody, Sieglinde, Silverton Russet, Sirco, Snowden, Spunta, Up to date, Stobrawa, Superior, Vivaldi, Vitelotte, Yellow Finn, or Yukon Gold can be used to obtain a potato polysaccharide preparation provided herein.

Any appropriate method can be used to obtain a potato polysaccharide preparation provided herein. For example, raw potato material can be homogenized (e.g., homogenized with a Polytron homogenizer) in water and maintained at room temperature for a period of time (e.g., about 1 hour) with occasional shaking. The homogenate can be centrifuged (e.g., centrifuged at 4000 g for 10 minutes) to remove any larger solid material. The resulting supernatant can be loaded onto a Solid Phase Extraction cartridge (e.g., a C18 cartridge such as a Sep-Pak Plus C-18 cartridge), and the polysaccharide material eluted with 10 percent acetonitrile. Once eluted, the polysaccharide material can be dried and stored (e.g., stored at about 4°C).

This document also provides nutritional supplement compositions containing one or more potato polysaccharide preparations provided herein. For example, a potato polysaccharide preparation provided herein obtained from Idaho Russet potatoes can be formulated into a nutritional supplement composition.

Any appropriate dose of a potato polysaccharide preparation provided herein can be used to formulate a composition provided herein (e.g., a nutritional supplement composition or potato polysaccharide preparation provided herein). For example, a potato polysaccharide preparation provided herein can be used to formulate a composition for reducing the number of cancer cells within a mammal having cancer cells that express a KRAS polypeptide. The composition can contain between about 1 mg and about 750 mg (e.g., between about 1 mg and about 500 mg, between about 1 mg and about 250 mg, between about 5 mg and about 40 mg, between about 5 mg and about 30 mg, between about 5 mg and about 20 mg, between about 6 mg and about 50 mg, between about 6 mg and about 20 mg, between about 10 mg and about 25 mg, or between about 15 mg and about 20 mg) of the potato polysaccharide component of the potato polysaccharide preparation. In some cases, a composition (e.g., a nutritional supplement composition) can be formulated to deliver about 0.05 mg of the potato polysaccharide component per kg of body weight to about 0.5 mg of the potato polysaccharide component per kg of body weight to a mammal (e.g., a human) per day. For example, a nutritional supplement composition can be formulated into a single oral composition that a human can swallow once a day to provide between about 0.05 mg of the potato polysaccharide component per kg of body weight to about 0.5 mg of the potato polysaccharide component per kg of body weight.

Any appropriate method can be used to formulate a composition provided herein (e.g., a nutritional supplement composition or potato polysaccharide preparation provided herein). For example, common formulation mixing techniques and preparation techniques can be used to make a composition (e.g., a nutritional supplement composition) having the components described herein. In addition, a composition provided herein (e.g., a nutritional supplement composition or potato polysaccharide preparation provided herein) can be in any form. For example, a composition provided herein (e.g., a nutritional supplement composition or potato polysaccharide preparation provided herein) can be formulated into a pill, capsule, tablet, gel cap, nutritional shake, nutritional bar, rectal suppository, sublingual suppository, nasal spray, inhalant, or injectable ampule. In some cases, a composition provided herein (e.g., a nutritional supplement composition) can include one or more potato polysaccharide preparations provided herein alone or in combination with other ingredients including, without limitation, gelatin, cellulose, starch, sugar, bentonite, lactic acid, mannitol, alpha lipoic acid, alpha tocopherol, L-ascorbate, or combinations thereof.

As described herein, a composition containing a potato polysaccharide preparation provided herein (e.g., a nutritional supplement composition or a potato polysaccharide preparation provided herein) can be used to increase or decrease expression of a KRAS polypeptide and/or a polypeptide involved with cancer. For example, a composition containing a potato polysaccharide preparation provided herein or a potato polysaccharide preparation provided herein can be used to decrease expression of a KRAS polypeptide, a SOC2 polypeptide, an ILK polypeptide, an HSP9A polypeptide, or a combination thereof.

In some cases, a composition provided herein can be used to decrease expression of a KRAS polypeptide by about 5 % to about 70 % (e.g., from about 10 % to about 70 %, from about 15 % to about 70 %, from about 20 % to about 70 %, from about 5 % to about 45 %, from about 5 % to about 60 %, from about 5 to about 50 %, from about 15 % to about 40 %, or from about 20 % to about 30 %). In some cases, a composition provided herein can be used to decrease expression of a SOC2 polypeptide by about 5 % to about 50 % (e.g., from about 10 % to about 50 %, from about 15 % to about 50 %, from about 20 % to about 50 %, from about 5 % to about 45 %, from about 5 % to about 40 %, from about 5 to about 35 %, from about 15 % to about 30 %, or from about 20 % to about 40 %). In some cases, a composition provided herein can be used to decrease expression of an ILK polypeptide by about 5 % to about 50 % (e.g., from about 10 % to about 50 %, from about 15 % to about 50 %, from about 20 % to about 50 %, from about 5 % to about 45 %, from about 5 % to about 40 %, from about 5 to about 35 %, from about 15 % to about 30 %, or from about 20 % to about 40 %). In some cases, a composition provided herein can be used to decrease expression of a HSPA9 polypeptide by about 5 % to about 50 % (e.g., from about 10 % to about 50 %, from about 15 % to about 50 %, from about 20 % to about 50 %, from about 5 % to about 45 %, from about 5 % to about 40 %, from about 5 to about 35 %, from about 15 % to about 30 %, or from about 20 % to about 40 %).

In humans, a composition containing a potato polysaccharide preparation provided herein or a potato polysaccharide preparation provided herein can be used to decrease expression of a human KRAS polypeptide, a human SOC2 polypeptide, a human ILK polypeptide, a human HSPA9 polypeptide, or a combination thereof.

A human KRAS polypeptide can have the amino acid sequence set forth in GenBank® Accession No. NP_203524.1 (GI No. 15718763) and can be encoded by the nucleic acid sequence set forth in GenBank® Accession No. NP_033360.2 (GI No. 34485724). In some cases, a human KRAS polypeptide can have the amino acid sequence set forth in GenBank® Accession No. NM_004976.2 (GI No. 15718761) and can be encoded by the nucleic acid sequence set forth in GenBank® Accession No. NM_004985.3 (GI No. 34485723). In some cases, a human KRAS polypeptide can have the amino acid sequence set forth in GenBank® Accession No. XP_005253422.1 (GI No. 530399133) and can be encoded by the nucleic acid sequence set forth in GenBank® Accession No. XM_005253365.1 (GINo. 530399132). A human SOC2 polypeptide can have the amino acid sequence set forth in GenBank® Accession No. NP_031399.2 (GI No. 41281398) and can be encoded by the nucleic acid sequence set forth in GenBank® Accession No. NM_001269039.1 (GI No. 392841223). A human ILK polypeptide can have the amino acid sequence set forth in GenBank® Accession No. CAG28601.1 (GI No. 47115283) and can be encoded by the nucleic acid sequence set forth in GenBank® Accession No. NM_001014794.2 (GI No. 510785737). A human HSP9A polypeptide, can have the amino acid sequence set forth in GenBank® Accession No. NP_004125.3 (GI No. 24234688) and can be encoded by the nucleic acid sequence set forth in GenBank® Accession No. NG_029469 (GINo. 340523104).

The document will provide addition description in the following examples, which do not limit the scope of the invention described in the claims.

### EXAMPLES

### Example 1 - Identification and characterization of a potato polysaccharide preparation

6 grams of a Russet potato variety of the *Solanum tuberosum* species were homogenized with a Polytron homogenizer in 20 mL water in a 50 mL centrifuge tube and kept at room temperature for 1 hour. The homogenate was centrifuged at 4000 rpm for 10 minutes. A Sep-Pak Plus C-18 cartridge was activated with 10 mL 100% acetonitrile (ACN) and washed with 10 mL 0.05% trifluoroacetic acid in water (TFA water). 10 mL of the supernatant was loaded onto the cartridge, and all H₂O that passes through cartridge was collected in 1.5 mL Eppendorf tubes. Next, 10 mL of 2% ACN (in 0.05%TFA water) was passed through the column, and the elutriate was collected in 1.5 mL Eppendorf tubes. Next, 10 mL of 5% ACN (in 0.05%TFA water) was used to wash the column, and the elutriate was collected in 1.5 mL Eppendorf tubes. Finally, 10 mL of 10% ACN (in 0.05% TFA water) was collected in 1.5 mL Eppendorf tubes after passing through the column. All of the fractions were dried, and the dried fractions of the same ACN concentration were reconstituted into 1 tube in 1 mL of 0.05% TFA water for further purification via HPLC or reconstituted in 1 mL of phosphate buffered saline for use in cell treatments.

A Waters 2695 separations module with a photodiode array detector was used to purify the 10% ACN extract. An XterraRP C18 column (4.6 X 150 mm) was used for the separation with 0.05% TFA water as the mobile phase. Each HPLC run was a 20 minute gradient ranging from 0 to 2.5% ACN. The injection volume was 100 µL, and the flow rate was 0.5 mL/minute. HPLC fractionation of the 10% ACN extract yielded three major UV absorbing peaks eluted at 3.5, 3.9, and 12.1 minutes (Figure 1). Collection and HPLC re-purification of the 3.5 minute fraction yielded a symmetrical peak displaying a maximum absorbance at 198.3 nm (Figure 2).

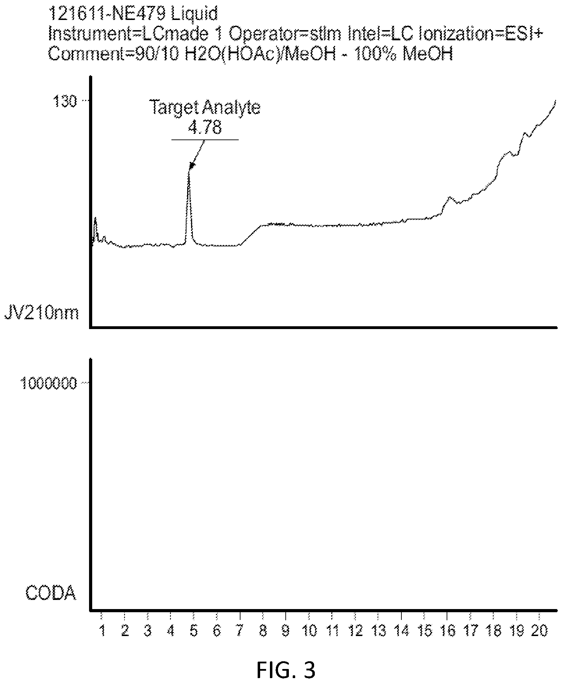

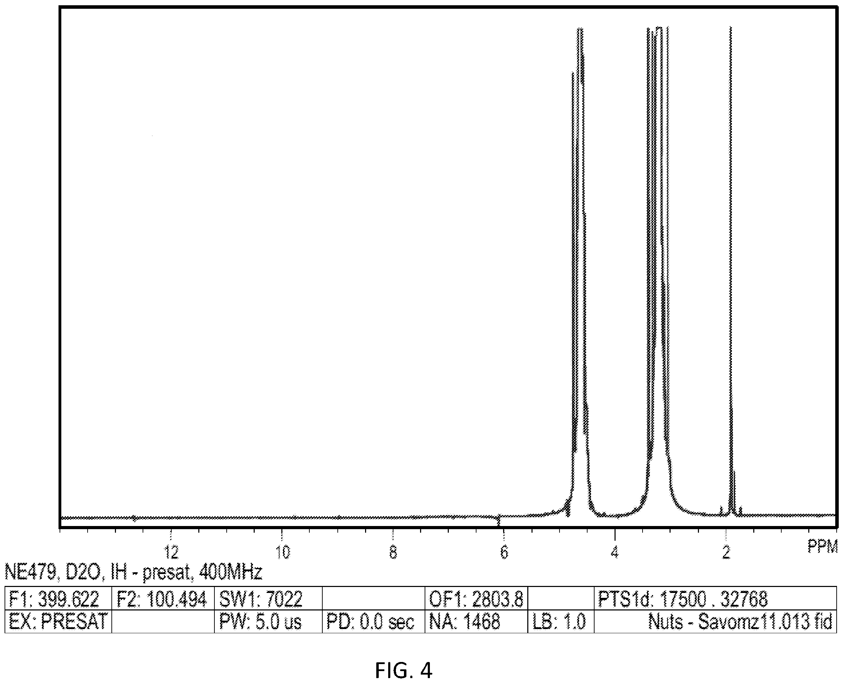

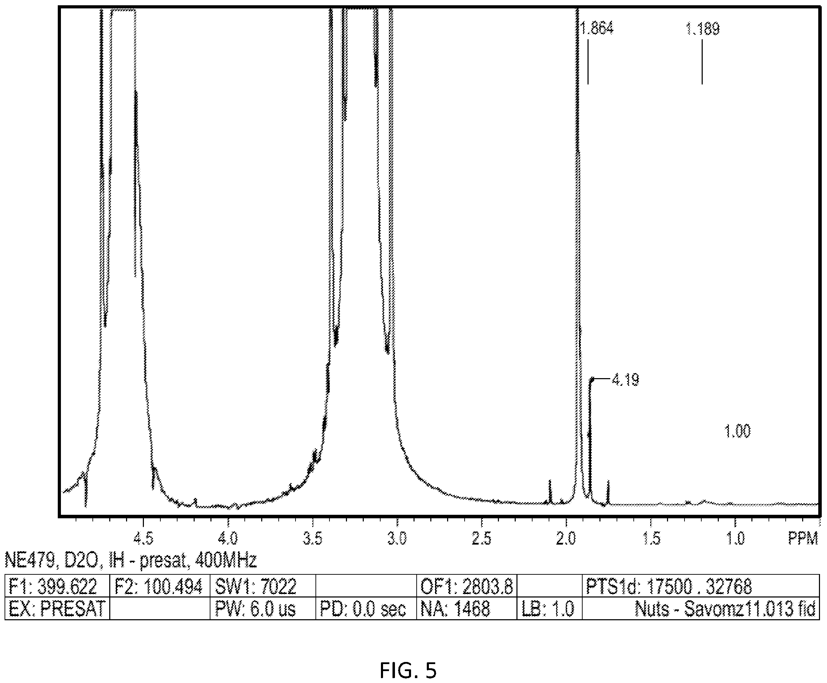

Further chemical characterization of the symmetrical 3.5 minute HPLC peak material was performed. Pooled 3.5 minute HPLC fractions were dried and reconstituted in 1 mL TFA water and subjected to tandem LC/MS/MS (Figure 3) and NMR chemical analyses (Figures 4 and 5). For the NMR analysis, ¹H-NMR was run on the sample using deuterium oxide (D₂O) as a solvent to further analyze the sample. The water peak at 4.65 PPM was solvent-suppressed, and the spectrum was acquired for several hours. Acetamide was detected at 3.2 PPM, along with acetonitrile at 1.9 PPM. Minor peaks were detected at 1.05 PPM, 1.17 PPM (broad peak), 1.189 PPM, and 1.864 PPM. One characteristic of polymeric materials in a proton NMR was the broadening of peaks such as the shift at 1.17 PPM. These shifts on the NMR could represent the peak at 4.8 PPM and suggested a polar, water-soluble polymer such as a polysaccharide. Taken together, these results confirmed the presence of high molecular weight polysaccharide material contained in HPLC purified fractions eluting at 3.5 minutes.

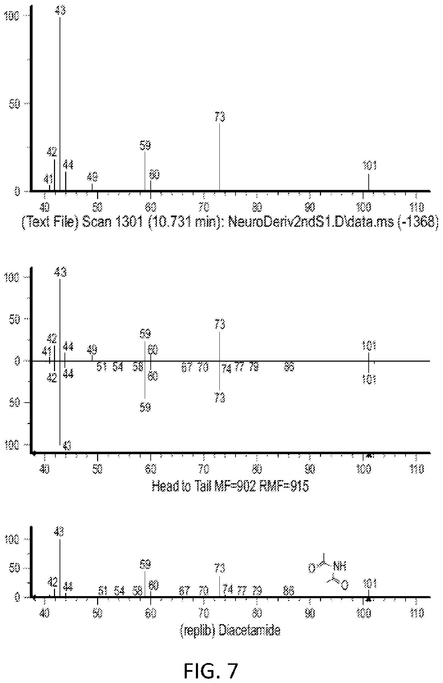

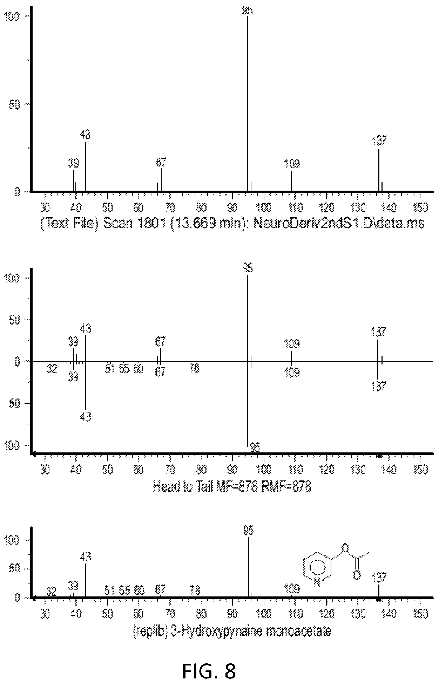

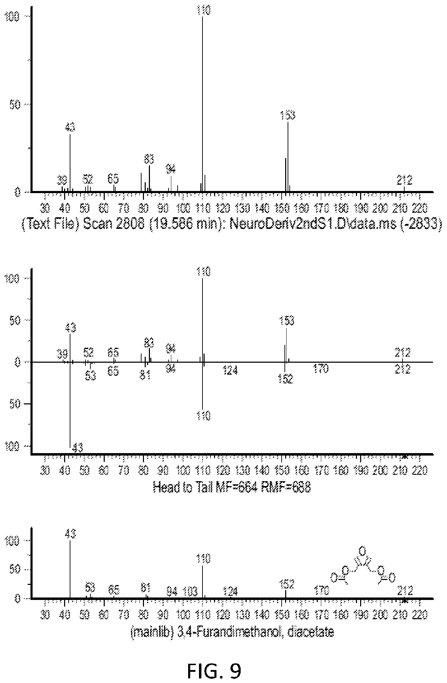

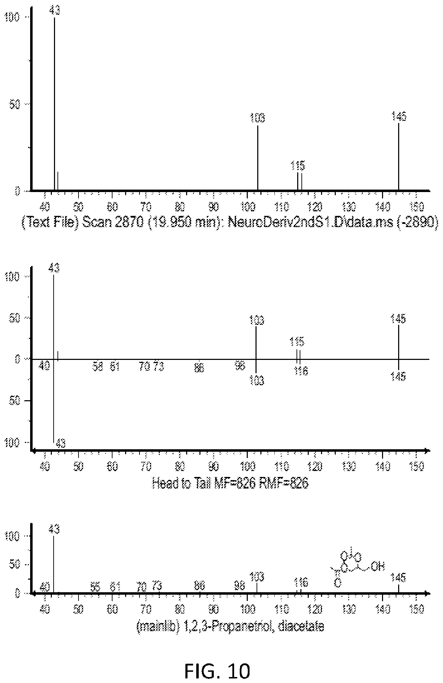

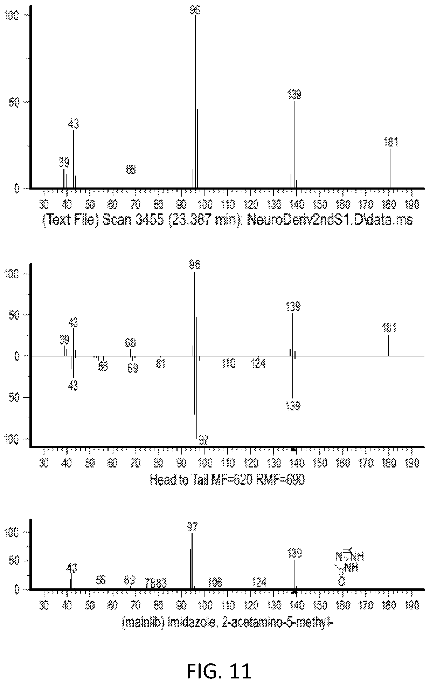

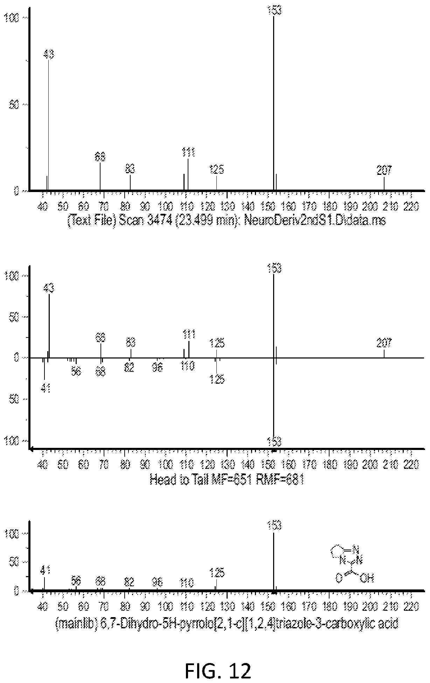

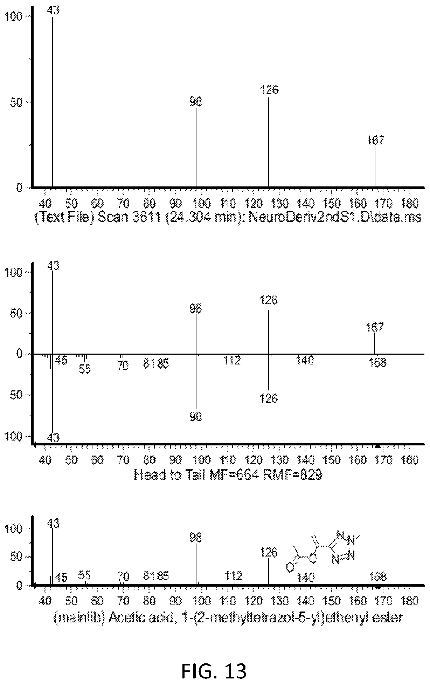

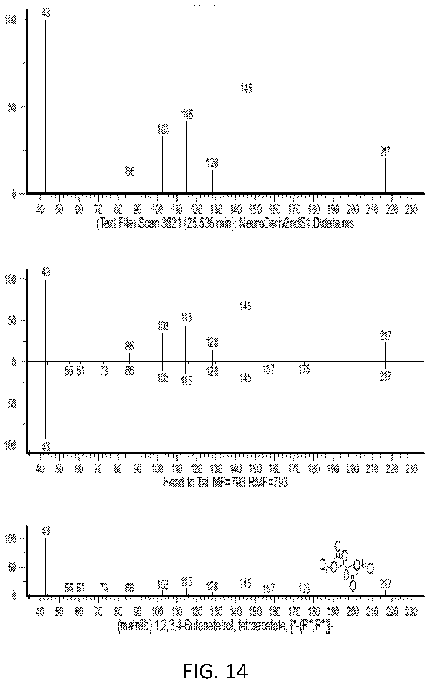

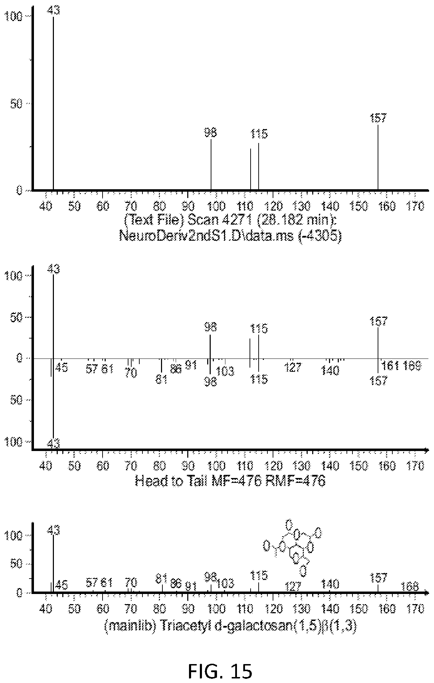

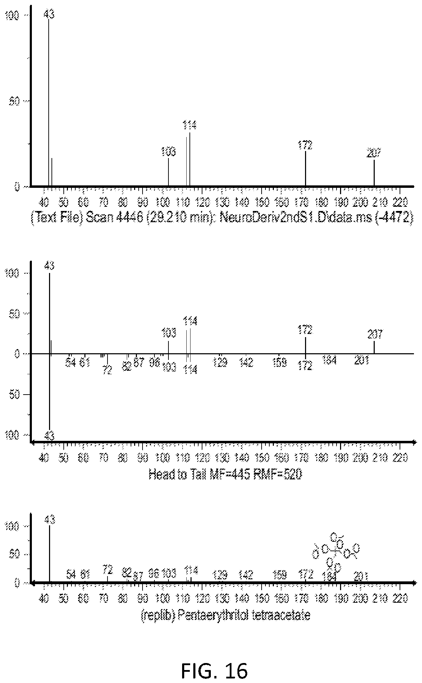

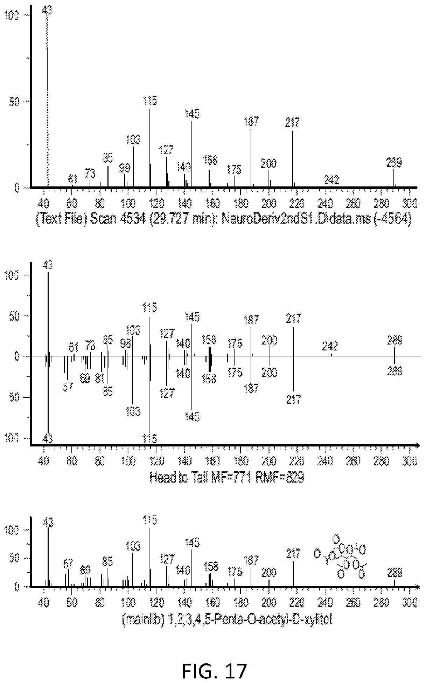

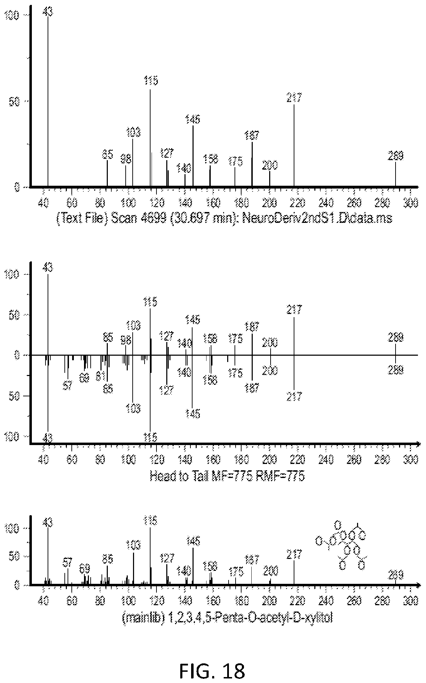

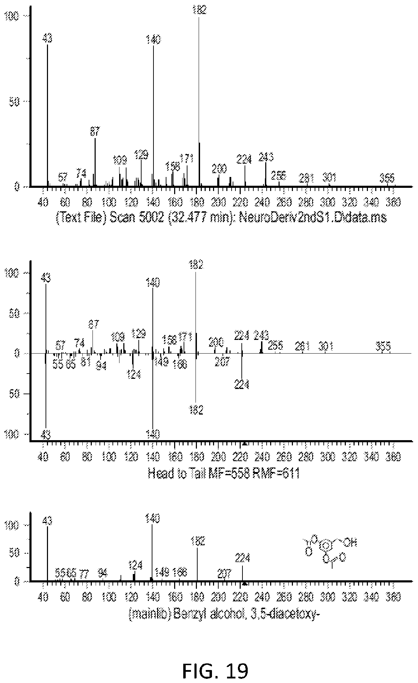

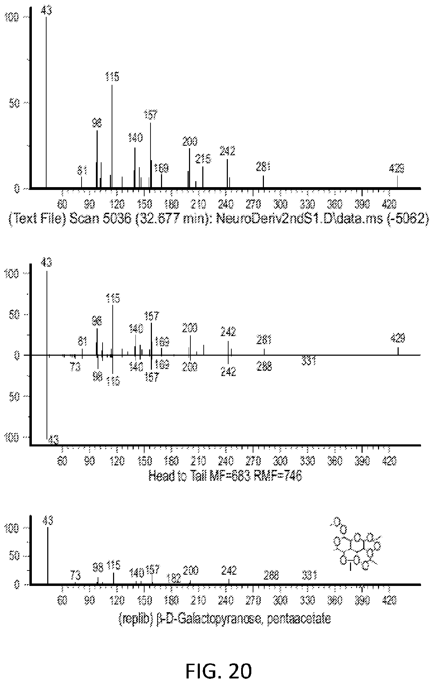

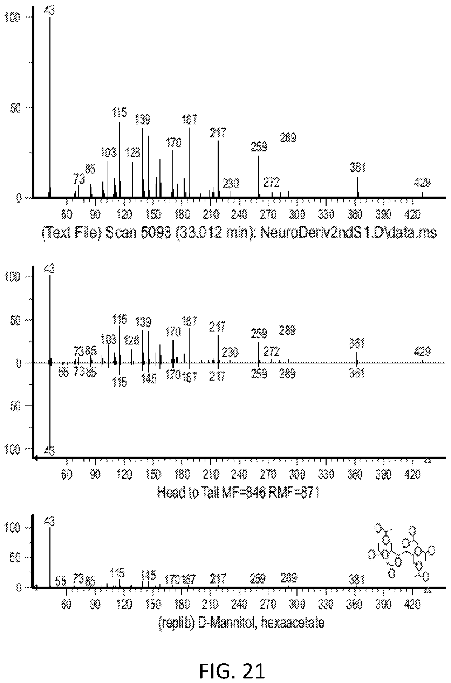

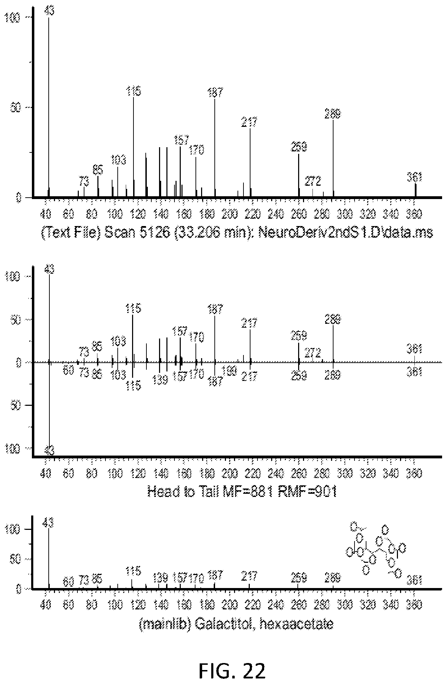

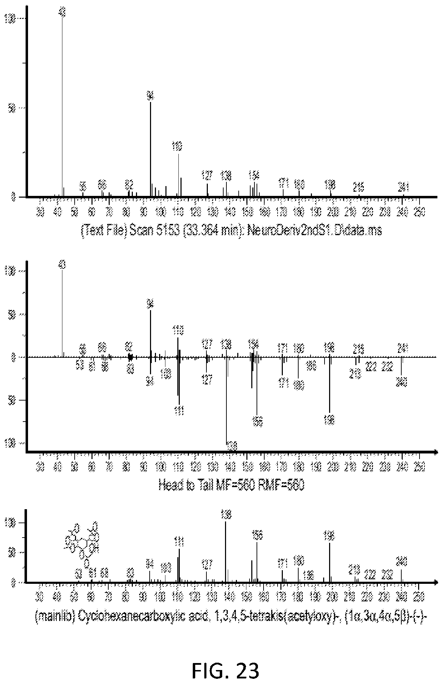

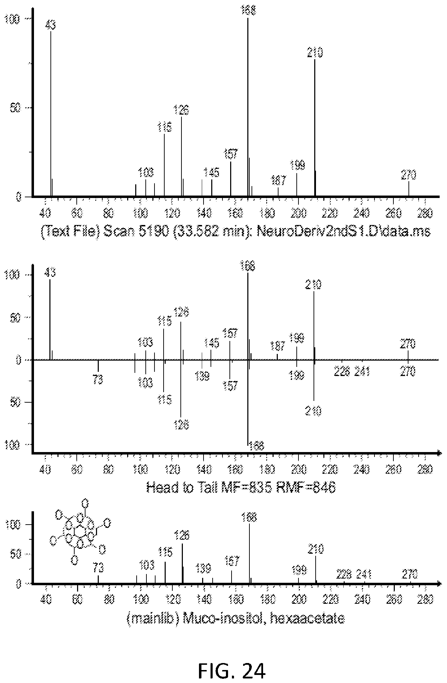

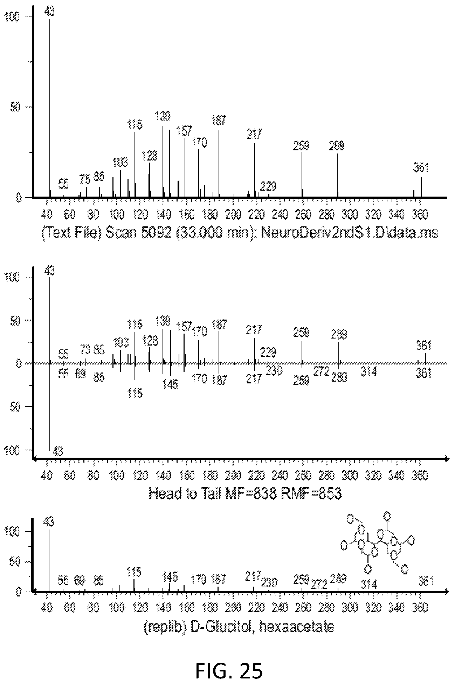

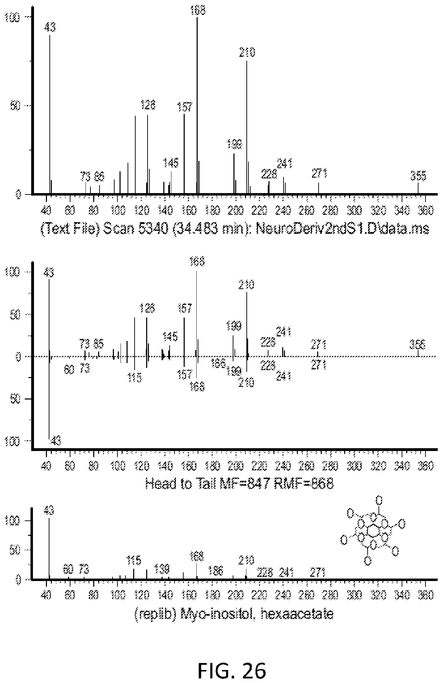

Further analysis confirmed that the HPLC purified fraction eluting at 3.5 minutes contains polysaccharide material (e.g., highly substituted complex xyloglucan material). To make the polysaccharide material analyzable by gas chromatography/mass spectroscopy (GC/MS), it was converted into its derivatized carbohydrate fragments. Briefly, the sample was concentrated to a dry residue that was hydrolyzed using trifluoroacetic acid. This was then reduced using sodium borohydride, and after borate removal, the end product was acylated using acetic anhydride and pyridine. The end products of the reaction were injected directly on GC/MS to identify any acylated carbohydrates. Based on the end analysis, a larger carbohydrate existed in the sample. The total ion chromatogram (TIC) is shown below in Figure 6 with appropriate peak labels below in Table 1. The major components identified are indicated in bold (peaks 3, 12, 14, and 21). The corresponding fragmentation for each compound is provided in Figures 7-26. For each fragmentation, the peak fragmentation pattern is on the top, the compound library fragmentation match is on the bottom, and an overlay of the two is in the center. Finally, unlabeled peaks were either column bleed or did not have a sufficient match to the compound library.

**Table 1: Summary of GC/MS results.**

| **Peak** | **Retention Time (min)** | **Compound Name** | **Structure** |
|---|---|---|---|
| 1 | 10.731 | Diacetamide | 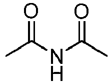 |
| 2 | 13.669 | 3-Acetoxy pyridine | 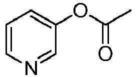 |
| 3 | **19.568** | **3,4-Furan dimethanol, diacetate** | 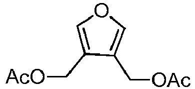 |
| 4 | 19.950 | 1,2,3-propanetriol diacetate | 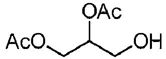 |
| 5 | 23.387 | Imidazole, 2-acetamino-5-methyl | 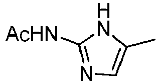 |
| 6 | 23.499 | 6,7-dihydro-5H-pyrrol[2,1,c][1,2,4] triazole-3-carboxylic acid | 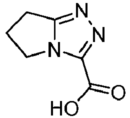 |
| 7 | 24.304 | Acetic acid, 1-(2-methyltetrazol-5-yl) ethenyl ester | 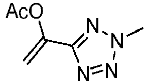 |
| 8 | 25.538 | 1,2,3,4-butanetriol, tetraacetate | 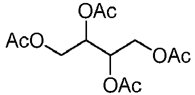 |
| 9 | 27.412 | (1,5)β(1,3)triacetyl D-galactosan (stereoisomer 1) | 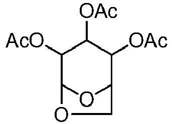 |
| 10 | 28.188 | (1,5)β(1,3)triacetyl D-galactosan (stereoisomer 2) | 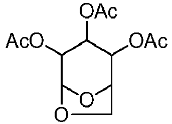 |
| 11 | 29.210 | Pentaerythritol tetraacetate | 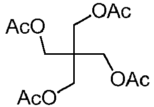 |
| **12** | **29.727** | **1,2,3,4,5-penta-o-acetyl-D-xylitol (isomer 1)** | 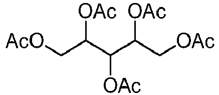 |
| 13 | 30.697 | 1,2,345-penta-o-acetyl-D-xylitol (isomer 2) | 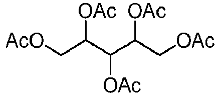 |
| **14** | **32.477** | **3,5-diacetoxy-benzyl alcohol** | 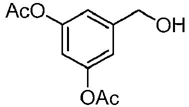 |
| 15 | 32.677 | β-D-glucopyranose, pentaacetate | 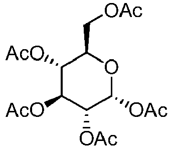 |
| 16 | 33.012 | D-mannitol hexaacetate | 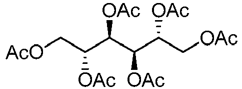 |
| 17 | 33.106 | β-D-galactopyranose, pentaacetate | 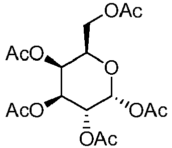 |
| 18 | 33.206 | Galacticol, hexaacetate | 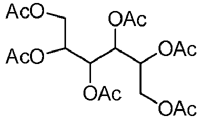 |
| 19 | 33.364 | Cyclohexane carboxylic acid, 1,2,45-tetrakis(acetoxy), (1α,3α,4α,5β)-(-) | 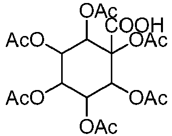 |
| 20 | 33.582 | Muco-inositol, hexaacetate | 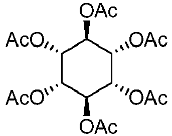 |
| **21** | **33.006** | **D-glucitol-hexaacetate** | 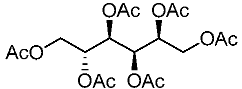 |
| 22 | 34.463 | Myo-inositol, hexaacetate | 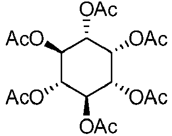 |

These results demonstrate the presence of sugar monomers that serve as building blocks for a larger carbohydrate. It appeared from these multiple lines of analysis that the potato polysaccharide preparation is a highly substituted complex xyloglucan.

### Example 2 - Sweet potatoes and multiple varieties of potatoes exhibit the presence of potato polysaccharide material

Six grams of potato material from multiple varieties of *Solanum tuberosum* (Organic yellow, Purple, Idaho Russet, and Yukon Gold) and six grams of material from sweet potatoes (*Ipomoea batatas*) were extracted in 20 mL of water. 10 mL of that water was then loaded onto a sep-pak cartridge, and the cartridge was then eluted with 10 mL of 10% ACN. The ACN was then dried, and the residue was dissolved in 1 mL of water. A 100 µL injection of this water was assessed using HPLC.

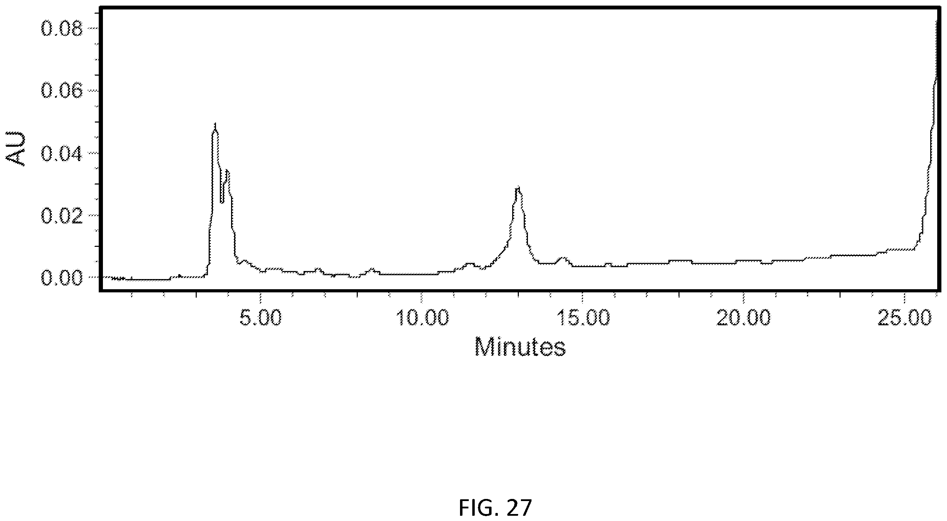

The HPLC chromatograms demonstrated that the amount of the first peak (at 3.5 minutes at 210 nm) was the same for all five types of potatoes tested (Figures 27-31).

In another experiment, material was extracted from a boiled Purple potato and analyzed. The peak at 3.5 minutes was not reduced in the boiled potato (Figure 32).

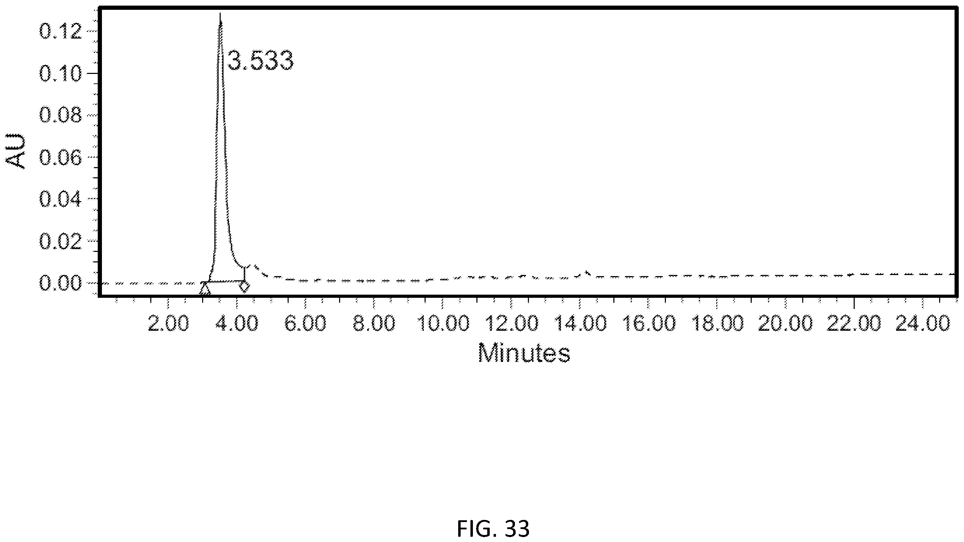

The 3.5 minute peak from two pooled fraction collections from Idaho Russet potatoes was collected, dried, and reconstituted in 100 µL of water. The material was then injected into the HPLC yielding a single peak at 3.5 minutes (Figure 33). Taken together, these results demonstrate that potatoes within the *Solanum tuberosum* and *Ipomoea batatas* species contain potato polysaccharide material.

### Example 3 - Analysis of a potato polysaccharide preparation

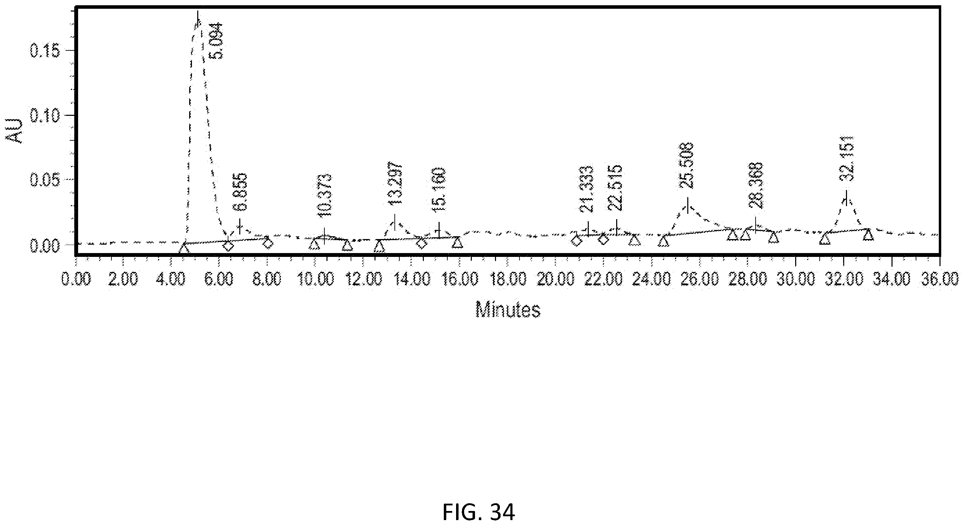

A potato polysaccharide preparation was purified using HPLC from 3 g of purple potato. The potato polysaccharide peak was eluted at about 5 minutes (Figure 34). This peak was obtained using a different chromatographic column (10 mm x150 mm) as compared to the column used to obtain the 3.5 minute peak. Since the column was a larger preparative column and the flow rate was 1.5 mL/minute, the elution time of the potato polysaccharide was 5 minutes.

### Example 4 - In vitro administration of a potato polysaccharide preparation to a KRAS-expressing human neuroblastoma cell line

To determine the effects of potato polysaccharide preparation administration on the expression of oncogenic KRAS and potentially interactive oncogenes in an established neuroblastoma cell line, HTB-11 neuroblastoma cells obtained from American Type Culture Collection (ATCC) were plated at a concentration of 5 x 10⁵ cells/2 mL into each well of 6-well culture plates using standard culture media. In separate incubations, HTB-11 neuroblastoma cells were administered purified potato polysaccharide preparation at a final concentration of 60 µg/mL or potato polysaccharide preparation vehicle for 4 hours. In vitro potato polysaccharide preparation trials were performed in triplicate.

### Extraction and purification of a potato polysaccharide preparation

Typically, 6 g of potato were homogenized with a Polytron homogenizer in 20 mL water in a 50 mL centrifuge tube and kept at room temperature for 1 hour. The homogenate was centrifuged at 4000 rpm for 10 minutes and the supernatant fraction was reserved. 10 mL of the supernatant fraction was percolated through a Sep-Pak Plus C-18 cartridge previously activated with 10 mL 100% acetonitrile (ACN) followed by 10 mL 0.05% trifluoroacetic acid in water (TFA water). Following successive low ACN washes, semi-purified potato polysaccharide preparation was eluted in 10 mL 10% ACN in 0.05% TFA water. The eluent fraction was dried and reconstituted in 1 mL 0.05 % TFA water for further purification via HPLC.

The reconstituted 10 % ACN eluent fraction was subjected to HPLC purification utilizing a Waters Xterra RP C18 column (4.6X150 mm) and Waters 2695 separations module with a photodiode array detector. HPLC purification employed a shallow 20 minute gradient ranging from 0 to 2.5 % in 0.05 % TFA water at a flow rate of 0.5 mL/min. Collection and HPLC re-purification of a major 198nm UV absorbing peak at 3.5 minutes yielded a symmetrical HPLC peak containing highly purified potato polysaccharide preparation. The purified HPLC fraction was dried and reconstituted in phosphate buffered saline (PBS) for use in biological experiments.

### RNA isolation

Following incubation of HTB-11 neuroblastoma cells with purified potato polysaccharide preparation, total RNA was isolated and purified using the RNeasy mini kit (Qiagen, Valencia, CA). Briefly, pelleted cells were re-suspended in 600 µL of RLT lysis buffer (Qiagen) and homogenized by passing the lysate 20 times through a 1 mL pipette tip. The samples were then processed according to the manufacturer's instructions (Qiagen, Valencia, Ca). In the final step, the RNA was eluted with 50 µL of RNase-free water by centrifugation for 1 minute at 13,000 g. The RNA was analyzed on a model 2100 bioanalyzer (Agilent, Santa Clara, CA) using a total RNA nanochip according to the manufacturer's protocol.

### DNA microarray analyses

DNA microarray analyses were performed using a system provided by Agilent. Arrays included four arrays per chip (Agilent 4X44K chips). Total RNA was reverse transcribed (400 ng) using T7 primers and labeled and transcribed using Cyanine-3 dye. Each array was hybridized with at least 1.65 µg of labeled cRNA at 65°C for 18 hours. Arrays were scanned using an Agilent array scanner. Array images were extracted with Agilent feature extraction software, and gene expression changes were calculated using Genespring version 12.6.

### Results

In vitro 4 hour administration of purified potato polysaccharide preparation to HTB-11 neuroblastoma cells engendered a statistically significant 21.4±8.0 % reduction of oncogenic KRAS gene expression, as monitored by DNA microarray analyses. Additionally, coordinate reductions of interactive SHOC2 and ILK oncogene expression of 16.7±3.1 % and 23.9±2.5 %, respectively, were observed as depicted in Table 2. The SHOC2 gene product is a cellular scaffold protein having repetitive leucine rich repeats that putatively link KRAS/ERK/MAP kinase signaling cascades. Coordinately dysregulated oncogenic KRAS and SHOC2 expression may override normative regulation of ERK1/2 activation by the epidermal EGFR-mediated signaling in major classes of human cancers. Additionally, KRAS and SHOC2 mutations have been linked to the development of Noonan Syndrome, an autosomal dominant condition leading to hematological malignancies and specific neuroblastoma and embryonal rhabdomyosarcoma solid tumors. Oncogenic ILK is an intracellular integrin:actin-bridging protein that is functionally linked to proliferation and metastatic outgrowth of primary tumor cells. Accordingly, the case for coordinate functional recruitment of oncogenic KRAS, SHOC2, and ILK in tumor growth, survival, and metastasis exists, demonstrating that a potato polysaccharide preparation can be used to treat cancers (e.g., human cancers) alone.

**Table 2. Diminished expression of oncogenic KRAS, SHOC2, and ILK oncogenes by in vitro potato polysaccharide preparation administration. P values are <0.05. Data sets were derived by DNA microarray analyses as described above.**

| **Gene symbol** | **Gene name** | **Percent reduction** |
|---|---|---|
| KRAS | Kirsten rat sarcoma viral oncogene homolog | 21.4±8.0 |
| | Transcript b | |
| | mRNA | |
| | NM_ 004985.3 GI:34485723 | |
| | Protein | |
| | NP_004976.2 GI:15718761 | |
| | Transcript X1 | |
| | mRNA | |
| | XM_ 005253365.1 GI:530399132 | |
| | Protein | |
| | XP_005253422.1 GI:530399133 | |
| | Transcript a | |
| | mRNA | |
| | NM_ 033360.2 GI:34485724 | |
| | Protein | |
| | NP_ 203524.1 GI:15718763 | |
| SHOC2 | soc-2 suppressor of clear homolog | 16.7±3.1 |
| | mRNA | |
| | NM_001269039.1 GI:392841223 | |
| | Protein | |
| | NP_ 031399.2 GI:41281398 | |
| ILK | integrin linked kinase | 23.9±2.5 |
| | mRNA | |
| | NM_001014794.2 GI:510785737 | |
| | Protein | |
| | CAG28601.1 GI:47115283 | |

These results also demonstrate that potato polysaccharide preparations can be used as anti-proliferative agents against cancer cells expressing a KRAS polypeptide.

### Example 5 - In vivo administration of a potato polysaccharide preparation to a genetically obese zucker zdf rat model

To determine the effects of potato polysaccharide preparation administration on the expression of oncogenic KRAS and potentially interactive oncogenes in the compromised livers of genetically obese Zucker ZDF rats.

### Experimental animals

Twenty-two 7-week old, male Zucker Diabetic Fatty rats (ZDF, Code: 370) and twenty-two 7-8 week old, male ZDF Lean rats (Code: 371) were purchased from Charles Rivers Laboratories (Wilmington, MA). The study animals were allowed an acclimation period of 4 days prior to baseline blood collections, at which time two extra animals from each strain were dropped from the study based on baseline body weight. The rats were housed two per cage and maintained in the Innovive caging system (San Diego, CA) upon arrival at PhysioGenix, Inc. Cages were monitored daily to ensure the Innovive system maintained 80 air changes per hour and positive pressure. In accordance with the Guide for Care and Use of Laboratory Animals (Eighth Edition), rat rooms were maintained at temperatures of 66-75 degrees Fahrenheit and relative humidity between 30 % and 70 %. The rooms were lit by artificial light for 12 hours each day (7:00 AM - 7:00 PM). Animals had free access to water and Purina 5008 rodent food (Waldschimdt's, Madison, WI) for the duration of the study except during fasted experiments.

### Potato polysaccharide preparation formulation

Purified potato polysaccharide preparation (10 mL stock solution at 5 mg/mL concentration) was stored at 4°C. The vehicle for the study was sterile water (Catalog number 002488, Butler Schein). Each week, the stock solution was diluted 1:100 in sterile water (0.05 mg/mL) and dispensed into daily aliquots. All vehicle and drug solutions were stored at 4°C and administered at room temperature daily by oral gavage (PO) in a volume of 1 mL/animal (0.15 mg/kg dose based on estimated body weight of 350 g).

### Dosing and grouping

Two types of rats were used for the study: homozygous obese ZDF/ZDF and heterozygous lean littermates. The rats within the groups were then chosen at random and divided into groups of 10. Group 1 was the homozygous ZDF/ZDF vehicle fed rats, group 2 was the homozygous ZDF/ZDF potato polysaccharide preparation fed, group 3 was the lean vehicle fed rat, and group 4 was the lean potato polysaccharide preparation fed rats. The vehicle was distilled water, and the potato polysaccharide preparation was given daily each morning via oral gavage at a dosage of 0.05 mg per animal. The dose was usually given in 1ml of water. Rats were caged in groups and maintained in 12 hours light/12 hours dark (7 am-7pm). The study lasted for 28 days, and all animals were euthanized by isoflurane overdose and thoracotomy following the collection of fasted blood glucose data on Day 28 of the study. Blood was collected via descending vena cava. Liver and abdominal fat were collected and weighed, and a portion of the left lateral liver lobe and abdominal fat were placed into individual histology cassettes and snap frozen in liquid nitrogen. General pathological observations were recorded.

### Real-time PCR analyses

RNA was isolated from frozen liver tissues using the Qiagen mini kit as described previously. The tissues (100 mg tissue and 1.8 mL of lysis buffer) were homogenized using a Polytron homogenizer. After the RNA was purified, 1 µg of total RNA was reverse transcribed using Superscript III reverse transcriptase and random primers (Invitrogen). Real-time PCR was performed with KRAS, SHOC2, ILK, and HSP9a detector sets (Applied Biosystem). SRSF4 was used as a reference gene. Using 1 µL of cDNA per reaction, all samples were analyzed in triplicate. The Real-time PCR master mix included 25 µL 2x universal master mix, 2.5 µL 20x detector set (with the primer and probe), and 21.5 µL of water. PCR was performed in an Applied Biosystems 7500 sequence detection system. The thermocycler conditions included denaturation at 95°C for 15 seconds and annealing/extension at 60°C for 60 seconds. Forty cycles of PCR were preceded by 95°C for 10 minutes. The relative quantities of genes were found using the formula 2-ΔΔCt using the Applied Biosystems 7500 software.

### Results

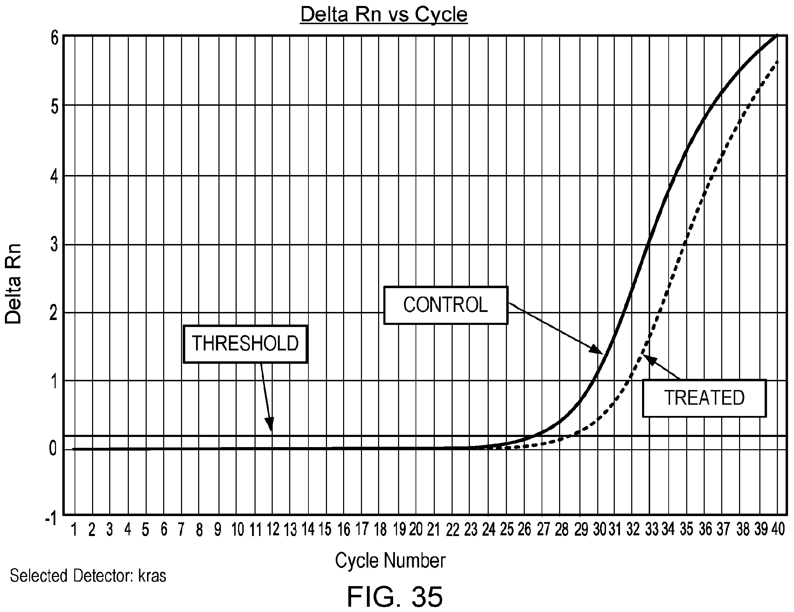

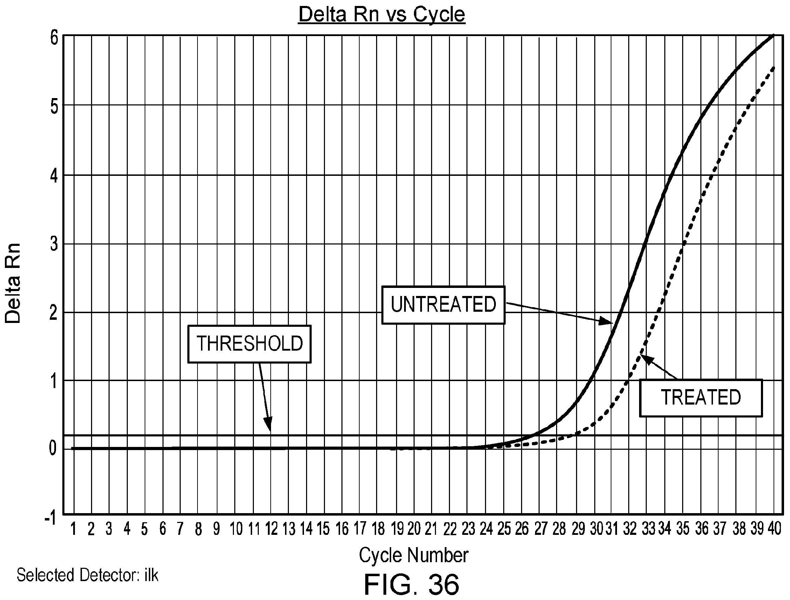

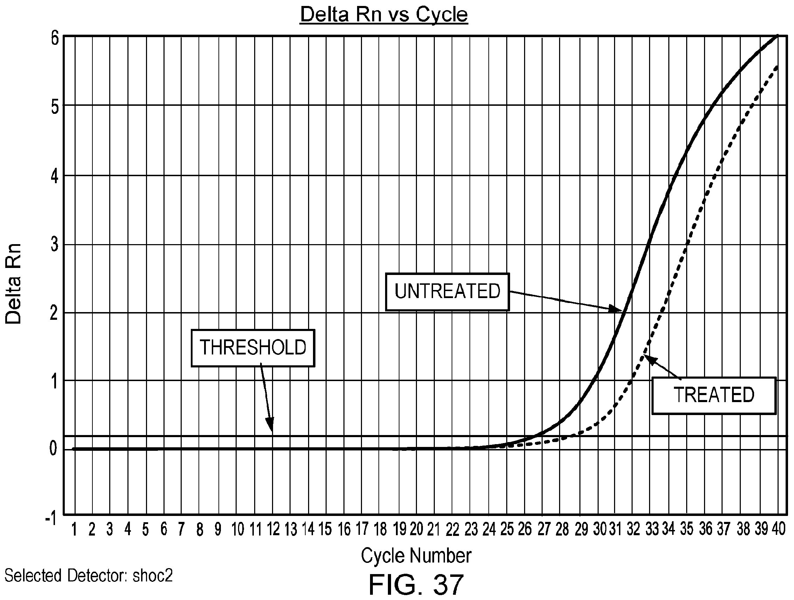

In vivo administration of purified potato polysaccharide preparation to ZDF rats (n=5) vs. vehicle control ZDF rats engendered a statistically significant 63.0±4.0% (p=0.01) reduction of oncogenic KRAS gene expression in compromised live tissues, as monitored by Real-time PCR analyses (Figure 35) and as depicted in Table 3. Additionally, coordinate statistically significant reductions of interactive SHOC2 (n=6) and ILK (n=6) oncogene expression of 27.9±1.6% (p=0.02) and 41.2±5.1% (p=0.01), respectively, were observed (Figure 36 and Figure 37). Thus, the inhibition of KRAS gene expression in concert with equivalent reductions in SHOC2 and ILK gene expression promoted by potato polysaccharide preparation administration demonstrates that a potato polysaccharide preparation provided herein can be used as an antineoplastic agent against cancer.

Furthermore, in vivo administration of purified potato polysaccharide preparation to heterozygous lean ZDF rat litter mates vs. vehicle control rats resulted in statistically significant decreases of 11.2±2.8 % and 26.2±2.5 % (p=0.04) in SHOC2 (n=6) and HSPA9 (n=6) oncogene expression, respectively, in liver tissues. These results demonstrate that a potato polysaccharide preparation provided herein can be used to reduce the risk of developing cancer.

**Table 3. Diminished expression of oncogenic KRAS, SHOC2, and ILK oncogenes in the livers of Zucker Diabetic Fatty rats following in vivo potato polysaccharide preparation administration. Data sets were derived by Real time PCR analyses, as described above.**

| **Gene symbol** | **Gene name** | **Percent reduction** |
|---|---|---|
| KRAS | Kirsten rat sarcoma viral oncogene homolog | 63.0±4.0% (p=0.01) |
| SHOC2 | soc-2 suppressor of clear homolog | 27.9±1.6% (p=0.02) |
| ILK | integrin linked kinase | 41.2±5.1% (p=0.01) |

## Claims

1. A composition for use in reducing the number of cancer cells in a mammal, wherein said composition consists essentially of a potato polysaccharide preparation obtained from raw potatoes, and
wherein said cancer cells are colorectal cancer cells, non-small-cell lung cancer cells, pancreatic cancer cells, liver cancer cells, or neuroblastoma cancer cells.

2. The composition for use according to claim 1, wherein said composition reduces expression of a KRAS polypeptide.

3. The composition for use according to claim 1, wherein said composition reduces expression of a SHOC2 polypeptide, an ILK polypeptide, or a HSP9A polypeptide.

4. The composition for use according to claim 1, wherein said mammal is a human.

5. The composition for use according to claim 1, wherein said composition consists essentially of said potato polysaccharide preparation in an amount that results in between 0.05 mg and 50 mg of the potato polysaccharide component of said potato polysaccharide preparation being administered to said mammal per kg of body weight of said mammal.

6. The composition for use according to claim 1, wherein said composition consists essentially of between 1 mg and 100 mg of said potato polysaccharide preparation, optionally wherein said composition consists essentially of between 6 mg and 20 mg of said potato polysaccharide preparation.

7. The composition for use according to claim 1, wherein said composition consists essentially of between 1 mg and 100 mg of the potato polysaccharide component of said potato polysaccharide preparation, optionally wherein said composition consists essentially of between 6 mg and 20 mg of the potato polysaccharide component of said potato polysaccharide preparation.

8. The composition for use according to claim 1, wherein said composition is in the form of a tablet.

9. The composition for use according to claim 1, wherein said potato polysaccharide preparation is in an amount that results in between 0.075 mg and 0.5 mg of the potato polysaccharide component of said potato polysaccharide preparation being administered to said mammal per kg of body weight of said mammal.

10. The composition for use according to claim 1, wherein at least about 80 percent of said potato polysaccharide preparation is potato polysaccharide, optionally wherein at least about 90 percent of said potato polysaccharide preparation is potato polysaccharide, optionally wherein at least about 95 percent of said potato polysaccharide preparation is potato polysaccharide.

## Patentansprüche

1. Zusammensetzung zur Verwendung beim Verringern der Anzahl an Krebszellen bei einem Säugetier,
wobei die Zusammensetzung im Wesentlichen aus einer Zubereitung von Kartoffelpolysacchariden besteht, die aus rohen Kartoffeln gewonnen wurde, und
wobei es sich bei den Krebszellen um Zellen des kolorektalen Karzinoms, Zellen des nichtkleinzelligen Lungenkarzinoms, Bauchspeicheldrûsenkrebszellen,
Leberkrebszellen oder Krebszellen des Neuroblastoms handelt.

2. Zusammensetzung zur Verwendung gemäß Anspruch 1, wobei die Zusammensetzung die Expression eines KRAS-Polypeptids verringert.

3. Zusammensetzung zur Verwendung gemäß Anspruch 1, wobei die Zusammensetzung die Expression eines SHOC2-Polypeptids, eines ILK-Polypeptids oder eines HSP9A-Polypeptids verringert.

4. Zusammensetzung zur Verwendung gemäß Anspruch 1, wobei es sich bei dem Säugetier um einen Menschen handelt.

5. Zusammensetzung zur Verwendung gemäß Anspruch 1, wobei die Zusammensetzung im Wesentlichen aus der Zubereitung von Kartoffelpolysacchariden in einer Menge besteht, die bewirkt, dass dem Säugetier zwischen 0,05 mg und 50 mg des Kartoffelpolysaccharidbestandteils der Zubereitung von Kartoffelpolysacchariden pro kg an Körpergewicht des Säugetiers verabreicht werden.

6. Zusammensetzung zur Verwendung gemäß Anspruch 1, wobei die Zusammensetzung im Wesentlichen aus 1 mg bis 100 mg der Zubereitung von Kartoffelpolysacchariden besteht, wobei die Zusammensetzung möglicherweise im Wesentlichen aus 6 mg bis 20 mg der Zubereitung von Kartoffelpolysacchariden besteht.

7. Zusammensetzung zur Verwendung gemäß Anspruch 1, wobei die Zusammensetzung im Wesentlichen aus 1 mg bis 100 mg des Kartoffelpolysaccharidbestandteils der Zubereitung von Kartoffelpolysacchariden besteht, wobei die Zusammensetzung möglicherweise im Wesentlichen aus 6 mg bis 20 mg des Kartoffelpolysaccharidbestandteils der Zubereitung von Kartoffelpolysacchariden besteht.

8. Zusammensetzung zur Verwendung gemäß Anspruch 1, wobei die Zusammensetzung einer in Form einer Tablette vorliegt.

9. Zusammensetzung zur Verwendung gemäß Anspruch 1, wobei die Zubereitung von Kartoffelpolysacchariden in einer Menge vorliegt, die bewirkt, dass dem Säugetier zwischen 0,075 mg und 0,5 mg des Kartoffelpolysaccharidbestandteils der Zubereitung von Kartoffelpolysacchariden pro kg an Körpergewicht des Säugetiers verabreicht werden.

10. Zusammensetzung zur Verwendung gemäß Anspruch 1, wobei Kartoffelpolysaccharide mindestens ungefähr 80 Prozent der Zubereitung von Kartoffelpolysachariden ausmachen, wobei Kartoffelpolysaccharide möglicherweise mindestens ungefähr 90 Prozent der Zubereitung von Kartoffelpolysachariden ausmachen, wobei Kartoffelpolysaccharide möglicherweise mindestens ungefähr 95 Prozent der Zubereitung von Kartoffelpolysachariden ausmachen.

## Revendications

1. Composition destinée à être utilisée en réduction du nombre de cellules cancéreuses chez un mammifère, ladite composition étant constituée essentiellement d'une préparation de polysaccharides de pomme de terre obtenue à partir de pommes de terre crues et
lesdites cellules cancéreuses étant des cellules de cancer colorectal, des cellules de cancer du poumon non à petites cellules, des cellules de cancer du pancréas,
des cellules de cancer du foie ou des cellules cancéreuses de neuroblastome.

2. Composition destinée à être utilisée selon la revendication 1, ladite composition réduisant l'expression d'un polypeptide KRAS.

3. Composition destinée à être utilisée selon la revendication 1, ladite composition réduisant l'expression d'un polypeptide SHOC2, d'un polypeptide ILK ou d'un polypeptide HSP9A.

4. Composition destinée à être utilisée selon la revendication 1, ledit mammifère étant un être humain.

5. Composition destinée à être utilisée selon la revendication 1, ladite composition étant constituée essentiellement de ladite préparation de polysaccharides de pomme de terre en une quantité qui résulte en ce qu'entre 0,05 mg et 50 mg du composant polysaccharides de pomme de terre de ladite préparation de polysaccharides de pomme de terre est administré audit mammifère par kg de poids corporel dudit mammifère.

6. Composition destinée à être utilisée selon la revendication 1, ladite composition étant constituée essentiellement d'entre 1 mg et 100 mg de ladite préparation de polysaccharides de pomme de terre, éventuellement ladite composition étant constituée essentiellement d'entre 6 mg et 20 mg de ladite préparation de polysaccharides de pomme de terre.

7. Composition destinée à être utilisée selon la revendication 1, ladite composition étant constituée essentiellement d'entre 1 mg et 100 mg du composant polysaccharides de pomme de terre de ladite préparation de polysaccharides de pomme de terre, éventuellement ladite composition étant constituée essentiellement d'entre 6 mg et 20 mg du composant polysaccharides de pomme de terre de ladite préparation de polysaccharides de pomme de terre.

8. Composition destinée à être utilisée selon la revendication 1, ladite composition étant sous la forme d'un comprimé.

9. Composition destinée à être utilisée selon la revendication 1, ladite préparation de polysaccharides de pomme de terre étant en une quantité qui résulte en ce qu'entre 0,075 mg et 0,5 mg du composant polysaccharides de pomme de terre de ladite préparation de polysaccharides de pomme de terre est administré audit mammifère par kg de poids corporel dudit mammifère.

10. Composition destinée à être utilisée selon la revendication 1, au moins environ 80 pour cent de ladite préparation de polysaccharides de pomme de terre étant des polysaccharides de pomme de terre, éventuellement au moins environ 90 pour cent de ladite préparation de polysaccharides de pomme de terre étant des polysaccharides de pomme de terre, éventuellement au moins environ 95 pour cent de ladite préparation de polysaccharides de pomme de terre étant des polysaccharides de pomme de terre.
